# EUROPEAN PATENT APPLICATION

(11) **EP 4 631 554 A1**
(43) Date of publication of application: **15.10.2025**
(21) Application number: 24169417.3
(22) Date of filing: 10.04.2024
(51) Int. Cl.: A61M 16/00

(54) **RESPIRATORY SUPPORT DEVICE, ACOUSTIC SENSOR SYSTEM, AND MEMBRANE**

(71) Applicant: Koninklijke Philips N.V., 5656 AG Eindhoven (NL)
(72) Inventor: DE VRIES, Jan Johannes Gerardus, Eindhoven (NL); DE GRAAF, Pascal, Eindhoven (NL)
(74) Representative: Philips Intellectual Property & Standards

(57) **Abstract**

There is provided a respiratory support device for providing pressurized air to a subject. The respiratory support device comprises a conduit, an acoustic sensor, a membrane, and a body. The conduit is for conveying an airflow. The conduit has an opening. The acoustic sensor is arranged outside the conduit. The membrane is arranged to cover the opening to separate the acoustic sensor and the airflow from each other. The body is attached to the membrane. The body is adapted to vibrate in response to sound propagating along the airflow. The acoustic sensor is adapted to generate a signal representative of the sound based on a vibration of the body.

## Description

### FIELD OF THE INVENTION

The invention relates to a respiratory support device for providing pressurized air to a subject. Further, the invention relates to an acoustic sensor system for use in the respiratory support device. Further, the invention relates to a membrane for use in the respiratory support device.

### BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers and people with conditions such as coronary artery disease, hypertension and diabetes mellitus.

To treat SDB conditions, use is made of respiratory support devices, such as positive airway pressure (PAP) devices. The respiratory support device provides pressurized air to a subject to keep the subject's airways open during sleep. The respiratory support device provides the pressurized air to a subject's nose and/or mouth via a patient interface. Known patient interfaces are a nasal pillow, an oronasal mask, or a full-face mask.

It was discovered that the sound propagating through the respiratory support device may provide information about the status of the respiratory support device. For example, PCT application WO2021/207796 discloses using a microphone to detect sounds travelling through a conduit. The detected sound is used to identify physical characteristics of the air circuit in the respiratory support device. The physical characteristics may relate to noise from the impeller, or noise from the motor of a blower, or other mechanical sounds. The acoustical sensor is arranged outside the conduit. The conduit has a port allowing sound to propagate from the conduit to the acoustical sensor. A sound permeable membrane is arranged to cover the port to separate the microphone from the airflow through the conduit. The membrane prevents humidified and/or contaminated air from reaching the microphone and protects the electronics of the microphone from moisture or contaminated air, such as air containing human bodily fluids such as mucus.

### SUMMARY OF THE INVENTION

However, because the sound from the conduit travels from the conduit, via the membrane to the microphone, the sound received by the microphone has less intensity than when the sound was in the conduit. Also, the membrane may deform the sound. As a result, the signal generated by the microphone represents the sound in the conduit with a limited accuracy.

It is an objective of the invention to provide a signal which more accurately represents a sound in the conduit, generated by an acoustic sensor arranged outside the conduit.

According to a first specific aspect, there is provided a respiratory support device for providing pressurized air to a subject. The respiratory support device comprises a conduit, an acoustic sensor, a membrane, and a body. The conduit is for conveying an airflow. The conduit has an opening. The acoustic sensor is arranged outside the conduit. The membrane is arranged to cover the opening to separate the acoustic sensor and the airflow from each other. The body is attached to the membrane. The body is adapted to vibrate in response to sound propagating along the airflow. The acoustic sensor is adapted to generate a signal representative of the sound based on a vibration of the body.

The membrane separates the acoustic sensor and the airflow from each other, preventing the airflow from contaminating the acoustic sensor. In addition or alternatively, the membrane prevents particles, such as volatile organic components, from the acoustic sensor or from any electronic component near the acoustic sensor. Such particles could contaminate the airflow. Because the membrane covers the opening, one side of the membrane is in contact with the airflow. As a result, sound propagating along the airflow causes the membrane to vibrate. Because the body is attached to the membrane, the body vibrates in response to the sound propagating along the airflow. Because the acoustic sensor is adapted to generate the signal representative of the sound based on a vibration of the body, the sound is attenuated less compared to the prior art. Sound propagating to the acoustic sensor does not need to be transmitted from the membrane to air, and from air to the microphone as in the prior art. Instead, according to the invention, there is an improved transmission of the sound from the membrane to the body attached to the membrane. The sound is directly received by the acoustic sensor via the body. This way, the acoustic sensor is able to provide the signal which more accurately represents sound in the conduit. For example, the body is arranged in the opening, such as in the center of the opening. For example, the body extends perpendicular to a main plane of the membrane. The membrane is adapted to, in response to the sound propagating along the airflow, vibrate with an amplitude that is larger in a direction perpendicular to the main plane than in a direction along the main plane. For example, the membrane has dimensions in the main plane that are substantially larger than a dimension in a direction perpendicular to the main plane. The dimensions of the membrane in the main plane are, for example, a length or a width or a diameter of the membrane. The dimension of the membrane perpendicular to the main plane is, for example, a thickness. For example, the dimensions of the membrane in the main are 5 times or 10 times larger than the dimension of the membrane perpendicular to the main plane. For example, the length of the membrane is 5 times or 10 times larger than the thickness.

For example, the respiratory support device is a positive airway pressure (PAP) device. A PAP device is adapted to provide pressurized air at a constant pressure to the subject. For example, the respiratory support device is a bi-level PAP (BiPAP) device. A BiPAP device is adapted to provide pressurized air at a first pressure during inhalation by the subject, and at a second pressure during exhalation by the subject. The first pressure is different from the second pressure. For example, the respiratory support device is an auto-PAP (APAP) device. An APAP device is adapted to provide the pressurized air to the subject in a range of pressures.

For example, the conduit is a tube or a hose. For example, the conduit is at least partly formed by a hollow section of the respiratory support device. For example, the conduit is formed by a bore inside the respiratory support device.

For example, the opening is a round opening or a square opening or a rectangular opening. For example, the opening is one of a plurality of openings grouped together.

For example, the membrane is a thin plate. For example, the membrane has a thickness and a length and a width. The thickness is at least 5 times, or at least 10 times smaller than the length and smaller than the width. For example, the membrane has a thickness and a diameter. The thickness is at least 5 times, or at least 10 times smaller than the diameter. For example, the thickness is in the range of 20 - 500 µm, for example in the range of 30 - 250 µm. For example, the diameter of the membrane is in the range of 1-20 mm, for example in the range of 5-10 mm. For example, the membrane comprises a metal or a plastic or a rubber or a polymer.

In an embodiment, the acoustic sensor is a microphone having a first part and a second part. The microphone is adapted to generate the signal based on a movement of the first part and the second part relative to each other. The body comprises the first part.

According to this embodiment, the first part of the microphone is attached to the membrane. As a result, vibrations of the membrane caused by the sound propagating along the airflow, directly cause movement of the first part of the microphone. This results in an improved detecting of the sound propagating along the airflow by the microphone. For example, the microphone is a dynamic microphone, or a condenser microphone, as is further described in further embodiments. In an embodiment, the microphone is a ribbon microphone. In this embodiment, the first part comprises a corrugated metal element, whereas the second part comprises a magnet. The corrugated metal element is arranged in a magnetic field of the magnet. Movement of the corrugated metal element through the magnetic field of the magnet, causes an electric signal representative of the movement of the corrugated metal element relative to the magnet. For example, the magnet is a permanent magnet or an electromagnet.

In an embodiment, the membrane comprises a protrusion adapted to attach the body to. The body is attached to the protrusion.

According to this embodiment, the protrusion is provided to form an interface to attach the body to the membrane. For example, the protrusion provides a surface to which the body is clamped, bonded, or glued. For example, the protrusion increases the thickness of the membrane locally. As a result of the increased thickness, the membrane deforms less at the location of the protrusion when the membrane vibrates due to the sounds propagating through the airflow. Because the membrane deforms less at the location of the protrusion, the connection between the body and the membrane is less stressed. Therefore, the connection is less likely to fail or fails after using the respiratory support device for a long time. As a result, the body is connected to the membrane with an improved lifetime. For example, the protrusion is located at the center of the membrane. For example, the protrusion is arranged in the opening, such as in the center in the opening. For example, the protrusion extends from the membrane in a direction of the acoustic sensor.

In an embodiment, the membrane comprises a silicone material. The protrusion comprises the silicone material.

According to this embodiment, silicone material is used to make the membrane. Silicone material has silicon has a low Young's modulus, allowing the sound to effectively vibrate the membrane. Also, silicone material is able to repel water and does not support microbiological growth. This helps to keep the membrane clean. Cleanliness is important because the membrane comes into contact with the airflow that is breathed by the subject. However, silicone material does not adhere well to many types of materials. Because both the membrane and the protrusion are made from silicone material, there is a strong bond between the membrane and the protrusion. The protrusion provides an additional surface to which the body is clamped, bonded or glued. The body is attached to the protrusion with a sufficiently strong connection by making use of the additional surface, despite the fact that silicone material does not adhere well to many types of materials. For example, the membrane and the protrusion are created as a single part. For example, the membrane and the protrusion are molded as a single part. The silicone material is a material that comprises silicone for the largest part. For example, more than 80% or more than 90% or more than 95% of a weight of the silicone material is caused by silicone. For example, the silicone material is obtained by combining the silicone with one or more other materials to obtain desirable properties of the silicone material.

In an embodiment, the protrusion forms a receptacle for receiving at least part of the first part.

According to this embodiment, the protrusion forms a receptacle, such as a cavity or a recess. The receptacle receives the first part. The first part is arranged at least partly in the receptacle. The receptacle provides a shape to connect with the first part. For example, the receptacle clamps the first part. For example, the first part is bonded or glued in the receptacle. For example, the receptacle has an opening allowing the first part to be inserted in the receptacle via the opening. For example, after the first part is inserted in the receptacle, a portion of the first part extends out of the receptacle via the opening. For example, the receptacle has no opening large enough to fit the first part through, or no opening at all. In this example, the protrusion is formed around the first part, for example by injection molding.

In an embodiment, the protrusion has a proximal part and a distal part. The proximal part has a different cross-section than the distal part. The first part is arranged around the proximal part.

According to this embodiment, the first part is arranged around the proximal part. For example, the first part is a ring-shaped magnet or a ring-shaped piece of metal, or an electrical coil. In an embodiment, the proximal part has a smaller cross-section than the distal part. In this embodiment, a main body of the membrane prevents the first part from moving away from the proximal part at one side of the proximal part. The main body of the membrane is the thin portion of the membrane extending substantially over the opening in the conduit in the main plane of the membrane. Because the distal part has a larger cross-section than the proximal part, the distal part prevents the first part from moving away from the proximal part via the distal part. As a result, the first part is attached to the protrusion by using the shapes of the proximal part and the distal part. Optional, the first part is additionally glued or bonded to the protrusion. For example, the dimensions and the elasticity of the distal part allow for the first part to be pressed along the distal part to the proximal part during assembly. When the first part is placed around the proximal part, the dimensions and the elasticity of the distal part prevent the first part from moving past the distal part during operational use. In another example, the first part comprises a coil that is wound around the proximal part. The coil has an inner diameter that is smaller than the outer diameter or the width of the distal part. In another embodiment, the proximal part has a larger cross-section than the distal part. This allows the first part to be assembled by moving the first part over the distal part. The distal part helps to align the first part relative to the proximal part. By moving the first part over the larger cross-section of the proximal part, the first part is clamped on the proximal part. For example, the cross-section of the proximal part is larger than an inner dimension of the first part. Optional, the first part is additionally glued or bonded to the proximal part.

In an embodiment the microphone is a dynamic microphone. The first part comprises one of a magnet and a coil. The second part comprises the other of a magnet and a coil. The magnet and the coil are arranged to cooperate electromagnetically with each other to generate an electrical signal in response to the movement of the first part and the second part relative to each other.

According to this embodiment, either the coil or the magnet of the dynamic microphone is the body attached to the membrane. In response to the sound propagating along the airflow, the membrane vibrates, and as a result the coil or magnet attached to the membrane vibrates. Because of these vibrations, the coil or the magnet attached to the membrane moves relative to the second part. The second part has the other of the coil or the magnet. Because of this relative movement, the coil generates an electrical signal representative of the relative movement. The electrical signal is or is part of the signal representative of the sound based on a vibration of the body. The electrical signal represents the sound propagating along the airflow more accurately, because the first part of the microphone is directly coupled to the membrane.

In an embodiment, the microphone is a condenser microphone. The first part comprises a first metal body. The second part comprises a second metal body. The first metal body and the second metal body form a capacitor having a capacitance. The condenser microphone is adapted to generate a change in capacitance in response to the movement of the first part and the second part relative to each other.

According to this embodiment, the sound propagating along the airflow causes the first metal body to vibrate. The vibration of the first metal body changes the distance between the first metal body and the second metal body. The change in distance results in a difference in capacitance between the first metal body and the second metal body. For example, the difference in capacitance is determined by applying a voltage over the first metal body and the second metal body. The difference in capacitance causes a difference in the voltage over the first metal body and the second metal body. The difference in the voltage is or is part of the signal representative of the sound based on a vibration of the body. The difference in the voltage represents the sound propagating along the airflow more accurately, because the first part of the microphone is directly coupled to the membrane. For example, the first metal body is a metallic layer arranged on the membrane. For example, the metallic layer is printed on the membrane.

In an embodiment, the acoustic sensor comprises a sensor membrane. The body extends from the membrane to the sensor membrane. The acoustic sensor is adapted to generate the signal representative of the sound based on a vibration of the sensor membrane.

According to this embodiment, the acoustic sensor has a sensor membrane. The acoustic sensor generates the signal based on the sound transmitted via the sensor membrane. By extending the body from the membrane to the sensor membrane, the membrane and the sensor membrane are coupled to each other via the body. Because of this coupling, vibrations of the membrane are transmitted via the body to the sensor membrane. As a result, the vibrations received by the sensor membrane more accurately correspond with the vibrations of the membrane. For example, the body is a rod or a tube or a strut. For example, the body is made from a material other than the membrane and the sensor membrane. For example, the body is made from a metal or a ceramic. By making the body from a metal or a ceramic, the body has a high stiffness, which improves the transmission of vibrations from the membrane to the sensor membrane. Preferably, the body has a high stiffness and a small mass to allow the transmission of high sound frequencies. For example, the membrane, the body, and the sensor membrane are made from the same material, such as silicone material. Because the membrane, the body and the sensor membrane are made from the same material, a strong bonding between the membrane and the body, and between the body and the sensor membrane can be made. For example, the membrane, the body, and the sensor membrane are made as a single part, for example by injection molding. For example, the membrane, the body, and the sensor membrane are made from the same material, wherein the body has a receptacle to receive an insert. The insert is made from a material with a higher Young's modulus than the material of the body. The insert improves the stiffness of the coupling between the membrane and the sensor membrane. For example, the insert is made from a metal or a ceramic. For example, the insert is a strut. For example, the acoustic sensor is a microphone having the sensor membrane. The membrane and the microphone are connected via the body. One side of the body is connected to the membrane, whereas another side of the body is connected to the sensor membrane of the microphone. For example, the microphone is a dynamic microphone or a condenser microphone.

In an embodiment, the body and the acoustic sensor are integrated. The acoustic sensor comprises an accelerometer.

According to this embodiment, the accelerometer is attached to the membrane. The sound propagating along the airflow vibrates the membrane. These vibrations cause the accelerometer to vibrate. As a result, the accelerometer provides an accurate measure of the sound propagating along the airflow. For example, one or more electrically conductive tracks are provided on the membrane to guide a signal representative of the acceleration of the accelerometer from the accelerometer towards a processing system. For example, the one or more electrically conductive tracks are printed on the membrane.

In an embodiment, the membrane comprises a base layer and a protective layer. The protective layer is chemically bonded to the base layer. The protective layer protects the base layer from humidity in the airflow.

According to this embodiment, the protective layer protects the base layer from humidity in the airflow. As a result, more materials are available for making the base layer. For example, paper-like materials are well-suited for making the base layer of the membrane. Paper-like materials can be used to make the base layer with a small thickness, while providing enough strength to withstand the pressure difference between both sides of the membrane. For example, the thickness is in the range of 20 - 500 µm, or in the range of 30 - 250 µm. The pressure at the side of the membrane facing the airflow may be substantially higher than the pressure at the other side of the membrane facing the acoustic sensor. For example, the airflow is pressurized, whereas the acoustic sensor is at an ambient pressure. The pressure difference between the pressurized airflow and the ambient pressure causes stress on the membrane.

In an embodiment, the respiratory support device comprises a seal and a casing.
The acoustic sensor is arranged inside the casing. The casing has a casing opening arranged to face the opening in the conduit. The membrane is attached to the casing. The seal is adapted to provide a sealing engagement between the conduit and the casing by abutting a surface of the conduit enclosing the opening and by abutting a surface of the casing enclosing the casing opening.

According to this embodiment, the casing holds the acoustic sensor and the membrane. This allows accurate or careful assembly of the acoustic sensor and the membrane with the casing. For example, specialized tools are used to arrange the membrane, the acoustic sensor, and the casing relative to each other. Such specialized tools may include a tool for gluing, a tool for bonding or a tool for accurately arranging the membrane relative to the casing, a tool for accurately arranging the acoustic sensor relative to the casing, and/or a tool for accurately arranging the acoustic sensor relative to the membrane. However, the assembly of the casing with the conduit can be done with ease, because only the casing opening is to be arranged to face the opening in the conduit, and the seal is provided into contact with the conduit and the casing to provide the sealing engagement. This way, assembly is simplified. For example, the casing has plastic or metal walls. For example, the casing has one or more removable panels. For example, one or more removable panels provide access to the inside of the casing to arrange the acoustic sensor inside the casing. For example, the membrane is clamped or bonded or glued to the casing. For example, the seal is made with an adhesive or a sealant. For example, the seal is a gasket or an O-ring. For example, the seal is arranged on an outer surface of the casing facing the conduit. In another example, the seal is arranged at least partly in a groove formed in an outer surface of the casing facing the conduit. For example, the seal is arranged on an outer surface of the conduit facing the casing. In another example, the seal is arranged at least partly in a groove formed in an outer surface of the conduit facing the casing. For example, the membrane is arranged to cover the casing opening.

In an embodiment, the respiratory support device comprises an air delivery tube and a water reservoir. The air delivery tube is adapted to deliver the pressurized airflow to a patient interface. The conduit is pneumatically connected to the air delivery tube. The conduit couples the water reservoir and the air delivery tube to each other.

According to this embodiment, the patient interface contacts the subject to provide the pressurized air to the subject. The patient interface is for example a nasal pillow, an oronasal mask, or a full-face mask. The air delivery tube delivers the pressurized airflow to the patient interface. The air delivery tube is a flexible tube to allow the subject to move while sleeping without being significantly hindered by the respiratory support device. The water reservoir is adapted to, for example, humidify the pressurized airflow. The conduit is pneumatically connected to the air delivery tube, and couples the water reservoir and the air delivery tube to each other. As a result, the conduit is located at an advantageous location to receive sounds from the subject via the air delivery tube, sounds from a pressure source of the respiratory support device that provides the pressurized airflow, and sounds from the water reservoir. These sounds may provide important information about the subject or information about a status of a component of the respiratory support device. Because the conduit is located at the advantageous location to receive the sounds, the acoustic sensor is able to properly receive the sounds via the opening in the conduit. As a result, the acoustic sensor is able to generate the signal to represent the information about the subject or about the status of a component more accurately. For example, the pressure source is an impeller or blower. For example, the component of the respiratory support device is an impeller, or a motor of a blower, or another mechanical component. For example, the sound that provides information about the subject comprises lung sounds or coughing or wheezing or snoring or airway sounds.

In an embodiment, the airflow is a flow of the pressurized air to the subject.

In an embodiment, the membrane comprises an electro-active polymer. The membrane is adapted to deform in response to sound propagating along the airflow. The electro-active polymer is adapted to generate an electric signal in response to a deformation of the membrane.

According to this embodiment, the electro-active polymer deforms in response to the sound propagating along the airflow vibrating the membrane. As a result of the deformation of the electro-active polymer, the electro-active polymer generates an electric signal. The electric signal is representative of the deformation of the electro-active polymer. As a result, the electric signal is representative of the sound propagating along the airflow. This way, additional information about the sound propagating along the airflow is obtained. Preferably, the electro-active polymer is a piezoelectric polymer. For example, the piezoelectric polymer is a polyvinylidene fluoride (PVDF), a poly(vinylidene fluoride-trifluoroethylene) (P(VDF-TrFE)), or a poly(vinylidene fluoride-trifluoroethylene-chlorofluoroethylene) (P(VDF-TrFE-CFE)). For example, the membrane is provided with the electro-active polymer on the surface of the membrane facing the airflow, on the surface of the membrane facing the acoustic sensor, or on both the surface of the membrane facing the airflow and the surface of the membrane facing the acoustic sensor.

In a second aspect of the invention, there is provided an acoustic sensor system for use in the respiratory support device according to the first aspect of the invention. The acoustic sensor system comprises the acoustic sensor, the membrane, the body attached to the membrane, and a casing. The acoustic sensor is arranged inside the casing. The casing has a casing opening arranged to face the opening in the conduit. The membrane is arranged to cover the casing opening.

In an embodiment, the acoustic sensor system comprises a seal. When in use in the respiratory support device according to the first aspect, the seal provides a sealing engagement between the conduit and the casing by abutting a surface of the conduit enclosing the opening and by abutting a surface of the casing enclosing the casing opening.

In a third aspect of the invention, there is provided a membrane comprising a protrusion for use in the respiratory support system according to the first aspect of the invention.

In a fourth aspect of the invention, there is provided a respiratory support device for providing pressurized air to a subject. The respiratory support device comprises a conduit, an acoustic sensor, and a membrane. The conduit is for conveying an airflow. The conduit has an opening. The acoustic sensor is arranged outside the conduit. The membrane is arranged to cover the opening to separate the acoustic sensor and the airflow from each other. The membrane comprises an electro-active polymer. The membrane is adapted to deform in response to sound propagating along the airflow. The electro-active polymer is adapted to generate an electric signal in response to a deformation of the membrane.

According to the fourth aspect, the electro-active polymer deforms in response to the sound propagating along the airflow vibrating the membrane. As a result of the deformation of the electro-active polymer, the electro-active polymer generates an electric signal. The electric signal is representative of the deformation of the electro-active polymer. As a result, the electric signal is representative of the sound propagating along the airflow. This way, accurate information about the sound propagating along the airflow is obtained. Preferably, the electro-active polymer is a piezoelectric polymer. For example, the piezoelectric polymer is a polyvinylidene fluoride (PVDF), a poly(vinylidene fluoride-trifluoroethylene) (P(VDF-TrFE)), or a poly(vinylidene fluoride-trifluoroethylene-chlorofluoroethylene) (P(VDF-TrFE-CFE)). For example, the membrane is provided with the electro-active polymer on the surface of the membrane facing the airflow, on the surface of the membrane facing the acoustic sensor, or on both the surface of the membrane facing the airflow and the surface of the membrane facing the acoustic sensor.

In an embodiment, the membrane comprises a base layer and a protective layer. The protective layer is chemically bonded to the base layer. The protective layer protects the base layer from humidity in the airflow.

According to this embodiment, the protective layer protects the base layer from humidity in the airflow. As a result, more materials are available for making the base layer. For example, paper-like materials are well-suited for making the base layer of the membrane. Paper-like materials can be used to make the base layer with a thickness, for example in the range of 20 - 500 µm, or in the range of 30 - 250 µm, while providing enough strength to withstand the pressure difference between both sides of the membrane. The pressure at the side of the membrane facing the airflow may be substantially higher than the pressure at the other side of the membrane facing the acoustic sensor. For example, the airflow is pressurized, whereas the acoustic sensor is at an ambient pressure. The pressure difference between the pressurized airflow and the ambient pressure causes stress on the membrane.

In an embodiment, the respiratory support device comprises an air delivery tube and a water reservoir. The air delivery tube is adapted to deliver the pressurized airflow to a patient interface. The conduit is pneumatically connected to the air delivery tube. The conduit couples the water reservoir and the air delivery tube to each other.

According to this embodiment, the patient interface contacts the subject to provide the pressurized air to the subject. The patient interface is for example a nasal pillow, an oronasal mask, or a full-face mask. The air delivery tube delivers the pressurized airflow to the patient interface. The air delivery tube is a flexible tube to allow the subject to move while sleeping without being significantly hindered by the respiratory support device. The water reservoir is adapted to, for example, humidify the pressurized airflow. The conduit is pneumatically connected to the air delivery tube, and couples the water reservoir and the air delivery tube to each other. As a result, the conduit is located at an advantageous location to receive sounds from the subject via the air delivery tube, sounds from a pressure source of the respiratory support device that provides the pressurized airflow, and sounds from the water reservoir. These sounds may provide important information about the subject or about a status of a component of the respiratory support device. Because the conduit is located at the advantageous location to receive the sounds, the acoustic sensor is able to properly receive the sounds via the opening in the conduit. As a result, the acoustic sensor is able to generate the signal to represent the information about the subject or about the status of a component more accurately.

In an embodiment, the respiratory support device comprises an accelerometer attached to the membrane. The accelerometer is adapted to vibrate in response to the sound propagating along the airflow. The accelerometer is adapted to generate a signal representative of the sound based on a vibration of the accelerometer.

According to this embodiment, the accelerometer is attached to the membrane. The sound propagating along the airflow vibrates the membrane. These vibrations cause the accelerometer to vibrate. As a result, the accelerometer provides an accurate measure of the sound propagating along the airflow. For example, one or more electrically conductive tracks are provided on the membrane to guide a signal representative of the acceleration of the accelerometer from the accelerometer towards a processing system. For example, the one or more electrically conductive tracks are printed on the membrane.

In an embodiment, the membrane comprises a silicone material.

According to this embodiment, silicone material is used to make the membrane. The silicone material has a low Young's modulus, allowing the sound to effectively vibrate the membrane. Also, silicone material is able to repel water and does not support microbiological growth. This helps to keep the membrane clean. Cleanliness is important because the membrane comes into contact with the airflow that is to breathe by the subject.

### BRIEF DESCRIPTION OF THE DRAWINGS

Exemplary embodiments will now be described, by way of example only, with reference to the following Figures, in which:
FIG. 1 depicts a respiratory support device according to a first embodiment of the invention;
FIG. 2 depicts a detail of the first embodiment;
FIG. 3 depicts a detail of a respiratory support device according to a second embodiment of the invention;
FIG. 4 depicts a detail of a respiratory support device according to a third embodiment of the invention;
FIG. 5 depicts a detail of a respiratory support device according to a fourth embodiment of the invention;
FIG. 6 depicts a detail of a respiratory support device according to a fifth embodiment of the invention;
FIG. 7 depicts a detail of a respiratory support device according to a sixth embodiment of the invention;
FIG. 8 depicts a detail of a respiratory support device according to a seventh embodiment of the invention;
FIG. 9 depicts a detail of a respiratory support device according to an eight embodiment of the invention;
FIG. 10 depicts a detail of a respiratory support device according to a nineth embodiment of the invention;

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying Figures. It should be understood that the Figures are merely schematic and are not drawn to scale. It should also be understood that the same reference numerals are used throughout the Figures to indicate the same or similar parts.

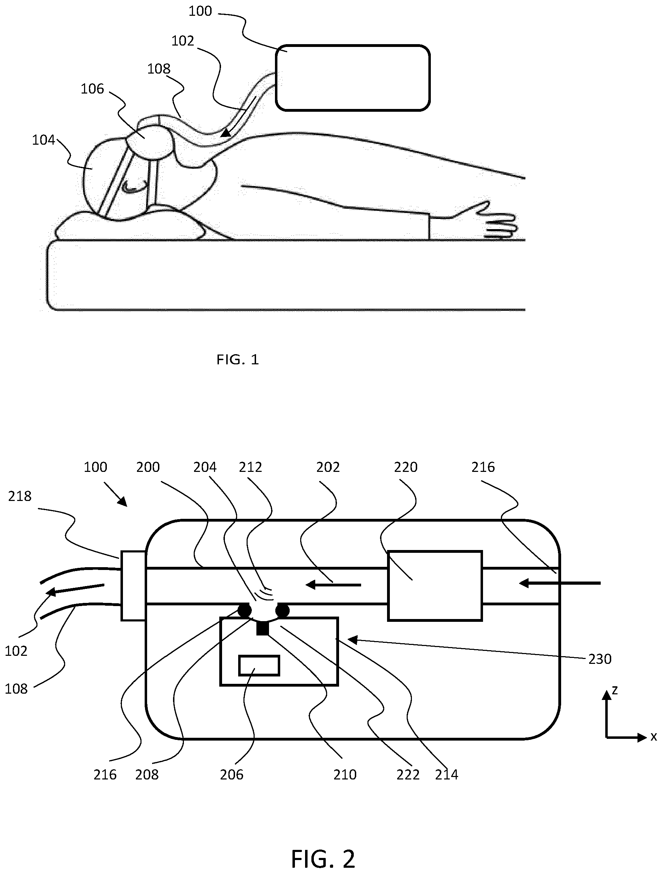

FIG. 1 depicts a respiratory support device 100 according to a first embodiment of the invention. The respiratory support device 100 is for providing pressurized air 102 to a subject 104. The respiratory support device 100 comprises an air delivery tube 108 connected to a patient interface 106. The air delivery tube 108 is adapted to deliver the pressurized air 102 to the patient interface 106. In this embodiment, the patient interface 106 is a face mask.

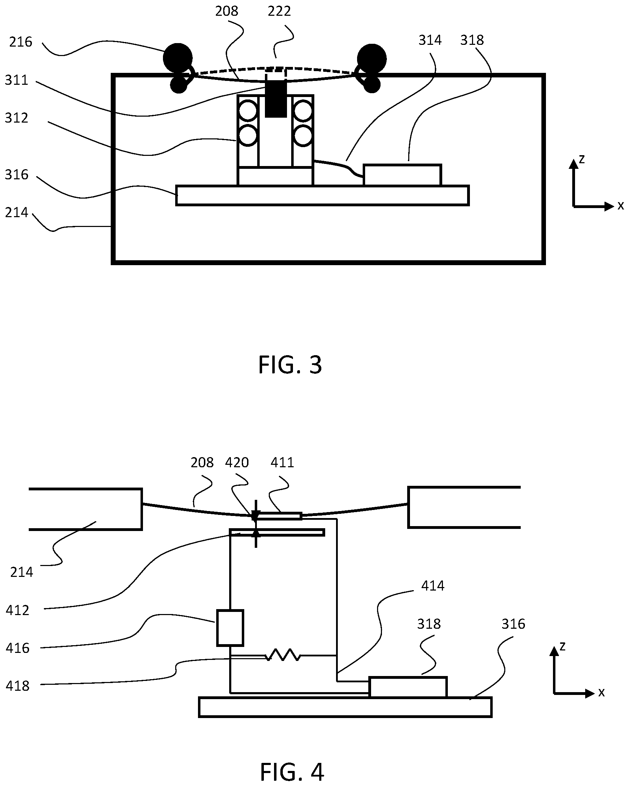

FIG. 2 depicts a detail of the first embodiment. The respiratory support device 100 comprises a conduit 200 for conveying an airflow 202. The conduit 200 has an opening 204. An acoustic sensor 206 is arranged outside the conduit 200. A membrane 208 is arranged to cover the opening 204 to separate the acoustic sensor 206 and the airflow 202 from each other. A body 210 is attached to the membrane 208. The body 210 is adapted to vibrate in response to sound 212 propagating along the airflow 202. The acoustic sensor 206 is adapted to generate a signal 314 representative of the sound 212 based on a vibration of the body 210. Signal 314 is depicted in FIG. 3.

The respiratory support device 100 comprises a connector 218 adapted to connect the air delivery tube 108 to the conduit 200. The respiratory support device 100 comprises a blower 220 adapted to generate the airflow 202 by pressuring air received from an inlet 216. In this embodiment, the airflow 202 is the flow of the pressurized air 102 to the subject 104. In another embodiment, the airflow 202 is air exhaled from the subject 104. In that embodiment, the airflow 202 is from the patient interface 106 towards an outlet to the ambient air.

The respiratory support device 100 comprises a seal 216 and a casing 214. The acoustic sensor 206 is arranged inside the casing 214. The casing 214 has a casing opening 222 arranged to face the opening 204 in the conduit 200. The membrane 208 is attached to the casing 214. The seal 216 is adapted to provide a sealing engagement between the conduit 200 and the casing 214 by abutting a surface of the conduit 200 enclosing the opening 204 and by abutting a surface of the casing 214 enclosing the casing opening 222.

The membrane 208 extends across the opening 204 in a main plane. The main plane is in the x-direction and the y-direction. Note that the y-direction is perpendicular to the x-direction and the z-direction depicted in FIG. 2. The membrane 208 is adapted to, in response to the sound 212 propagating along the airflow 202, vibrate with an amplitude that is larger in the direction perpendicular (i.e. along the z-direction) to the main plane than in a direction along the main plane. The body 210 extends perpendicular to the main plane of the membrane 208, i.e., extends in the negative z-direction from the membrane 208. The membrane 208 has dimensions in the main plane that are substantially larger than a dimension in the z-direction. The thickness of the membrane 208 is in the z-direction.

In FIG. 2, the membrane 208 and the opening 204 are arranged at a bottom surface of the conduit. In an alternative embodiment, the membrane 208 and the opening 204 are arranged at a top surface of the conduit. In a yet another alternative embodiment, the membrane 208 and the opening 204 are arranged at a side surface of the conduit.

An acoustic sensor system 230 for use in the respiratory support device 100 is formed. The acoustic sensor system 230 comprises the acoustic sensor 206, the membrane 208, the body 210 attached to the membrane 208, and the casing 214. The acoustic sensor 206 is arranged inside the casing 214. The casing 214 has a casing opening 222 arranged to face the opening 204 in the conduit 200. The membrane 208 is arranged to cover the casing opening 222.

Optionally, the acoustic sensor system 230 comprises the seal 216. When in use in the respiratory support device 100, the seal 216 provides a sealing engagement between the conduit 200 and the casing 214 by abutting a surface of the conduit 200 enclosing the opening 204 and by abutting a surface of the casing 214 enclosing the casing opening 222.

FIG. 3 depicts a detail of a respiratory support device 100 according to a second embodiment. The second embodiment has, for example, the same elements as the first embodiment, except for the following.

In the second embodiment, the acoustic sensor 206 is a microphone having a first part 311 and a second part 312. The microphone is adapted to generate the signal 314 based on a movement of the first part 311 and the second part 312 relative to each other. The body 210 comprises the first part 311.

The sound 212 propagating along the airflow 202 in the conduit 200 causes the membrane 208 to vibrate, as is indicated with the dashed lines in the FIG. 3. As a result of the vibrating membrane 208, the first part 311 moves relative to the second part 312. The movement of the first part 311 and the second part 312 relative to each other generates the signal 314. The signal 314 is transmitted to a processing system 318. The processing system 318 is configured to process the signal 314. For example, the processing system 318 is configured to determine a frequency or an intensity of the sound 212. For example, the processing system 318 is configured to implement an algorithm to classify the sound 212. For example, the processing system 318 is configured to implement an algorithm to derive information of the subject 104 from the sound 212. For example, the processing system 318 is configured to implement an algorithm to derive information of a component of the respiratory support device 100 from the sound 212.

Optionally, the acoustic sensor 206 and the processing system 318 are arranged on a base plate 316. The base plate 316 comprises, for example, a printed circuit board (PCB) onto which the acoustic sensor 206 is mounted.

In this embodiment, the microphone is a dynamic microphone. The first part 311 comprises one of a magnet and a coil, in this case the magnet. The second part 312 comprises the other of a magnet and a coil, in this case the coil. The magnet and the coil are arranged to cooperate electromagnetically with each other to generate an electrical signal, which is signal 314, in response to the movement of the first part 311 and the second part 312 relative to each other.

FIG. 4 depicts a detail of a respiratory support device 100 according to a third embodiment. The third embodiment has, for example, the same elements as the first embodiment, except for the following.

In the third embodiment, the acoustic sensor 206 is a microphone having a first part 311 and a second part 312. The microphone is adapted to generate the signal 414 based on a movement of the first part 311 and the second part 312 relative to each other. The body 210 comprises the first part 311.

The microphone is a condenser microphone. The first part 311 comprises a first metal body 411. The second part 312 comprises a second metal body 412. The first metal body 411 and the second metal body 412 form a capacitor having a capacitance. The condenser microphone is adapted to generate a change in capacitance in response to the movement of the first part 311 and the second part 312 relative to each other.

A battery 416 or any other type of electric power source is provided to generate a voltage over the first metal body 411 and the second metal body 412. The gap between the first metal body 411 and the second metal body 412 defines a capacitance between the first metal body 411 and the second metal body 412. When the membrane 208 vibrates in response to the sound 212 propagating along the airflow 202, the first metal body 411 moves relative to the second metal body 412. As a result of this movement, the size of the gap 420 changes. As a result of the change of the gap 420, the capacitance changes. The change in capacitance creates an electrical signal 414, which is transmitted to the processing system 318. Optionally, the electric circuit, which includes the first metal body 411, the second metal body 412 and the battery 416, includes a resistor 418.

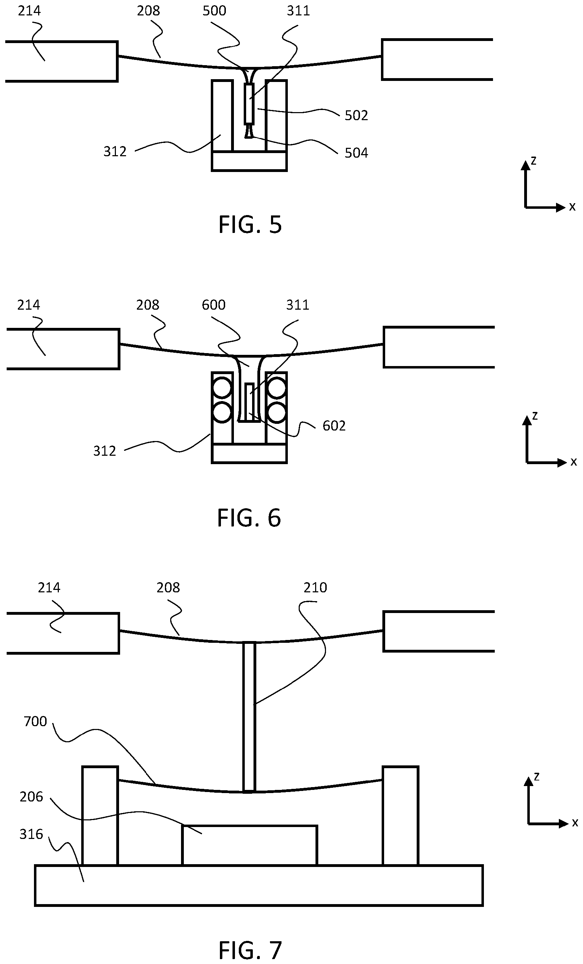

FIG. 5 depicts a detail of a respiratory support device 100 according to a fourth embodiment. The fourth embodiment has, for example, the same elements as the first embodiment, the second embodiment or the third embodiment, except for the following.

The membrane 208 comprises a protrusion 500 adapted to attach the body 210 to. The body 210 is attached to the protrusion 500.

The protrusion 500 has a proximal part 502 and a distal part 504. The proximal part 502 has a different cross-section than the distal part 504. The first part 311 is arranged around the proximal part 502.

In this embodiment, the proximal part 502 has a smaller cross-section than the distal part 504. In another embodiment, the proximal part 502 has a larger cross-section than the distal part 504.

Optionally, the membrane 208 comprises a silicone material. The protrusion 500 comprises the silicone material.

FIG. 6 depicts a detail of a respiratory support device 100 according to a fifth embodiment. The fifth embodiment has, for example, the same elements as the fourth embodiment, except for the following.

The membrane 208 comprises a protrusion 600 adapted to attach the body 210 to. The body 210 is attached to the protrusion 600. The protrusion 600 forms a receptacle 602 for receiving at least part of the first part 311.

FIG. 7 depicts a detail of a respiratory support device 100 according to a sixth embodiment. The sixth embodiment has, for example, the same elements as the previous embodiments, except for the following.

The acoustic sensor 206 comprises a sensor membrane 700. The body 210 extends from the membrane 208 to the sensor membrane 700. The acoustic sensor 206 is adapted to generate the signal 314 representative of the sound 212 based on a vibration of the sensor membrane 700.

In FIG. 7, the body 210 is depicted as a beam or strut. However, the body 210 is, for example, implemented in a different shape, such as an hour-glass shape or a round shape or an elliptical shape. For example, the length of the body 210 in the z-direction is as short as possible, but long enough to provide sufficient distance between the membrane 208 and the sensor membrane 700 to prevent the membrane 208 and the sensor membrane 700 to contact each other while vibrating. For example, the membrane 208 has the protrusion 500 or 600, wherein the body 210 is attached to the protrusion 500 or 600. For example, the sensor membrane 700 has a similar protrusion as protrusion 500 or 600, to which the body 210 is attached.

For example, the acoustic sensor 206 is a microphone having the sensor membrane 700. The membrane 208 and the microphone are connected via the body 210. One side of the body 210 is connected to the membrane 208, whereas another side of the body 210 is connected to the sensor membrane 700 of the microphone. For example, the microphone is a dynamic microphone or a condenser microphone.

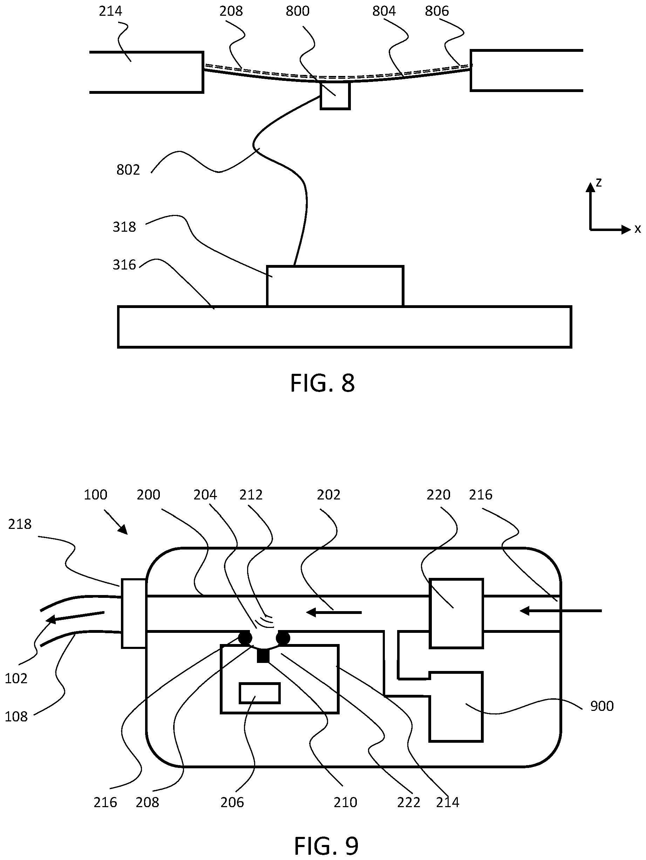

FIG. 8 depicts a detail of a respiratory support device 100 according to a seventh embodiment. The seventh embodiment has, for example, the same elements as the previous embodiments, except for the following.

The body 210 and the acoustic sensor 206 are integrated. The acoustic sensor 206 comprises the accelerometer 800.

According to this embodiment, the accelerometer 800 is attached to the membrane 208. The sound 212 propagating along the airflow 202 vibrates the membrane 208. These vibrations cause the accelerometer 800 to vibrate. As a result, the accelerometer 800 provides an accurate measure of the sound 212 propagating along the airflow 202. For example, one or more electrically conductive tracks are provided on the membrane 208 to guide a signal 802 representative of the acceleration of the accelerometer 800 from the accelerometer 800 towards the processing system 318. For example, the one or more electrically conductive tracks are printed on the membrane 208.

Further, this embodiment shows that the membrane 208 comprises a base layer 804 and a protective layer 806. The base layer 804 is indicated as a solid line, whereas the protective layer 806 is indicated as a double dashed line. The protective layer 806 is chemically bonded to the base layer 804. The protective layer 806 protects the base layer 804 from humidity in the airflow 202. Any of the embodiments described optionally has the base layer 804 and the protective layer 806.

FIG. 9 depicts a detail of a respiratory support device 100 according to an eighth embodiment. The eighth embodiment has, for example, the same elements as the previous embodiments, such as the first embodiment, except for the following.

The respiratory support device 100 comprises an air delivery tube 108 and a water reservoir 900. The air delivery tube 108 is adapted to deliver the pressurized air 102 flow to the patient interface 106. The conduit 200 is pneumatically connected to the air delivery tube 108. The conduit 200 couples the water reservoir 900 and the air delivery tube 108 to each other.

The water reservoir 900 is part of a humidifier. The humidifier is adapted to humidify air. The humidified air is provided to the subject 104 by the respiratory support device 100 to improve the comfort for the subject 104. For example, the humidifier is provided with a heating element to heat water in the water reservoir 900 to evaporate the water. By evaporating the water, the humidifier humidifies air.

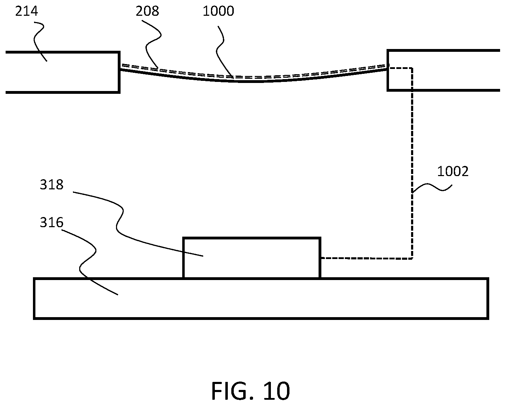

FIG. 10 depicts a detail of a respiratory support device 100 according to a nineth embodiment. The nineth embodiment is, for example, used in combination with any one of the first to the eight embodiments. For example, the nineth embodiment is not used in combination with any one of the first to the eight embodiments.

In the nineth embodiment, there is provided a respiratory support device 100 for providing pressurized air 102 to a subject 104. The respiratory support device 100 comprises a conduit 200, an acoustic sensor 206, and a membrane 208. The conduit 200 is for conveying an airflow 202. The conduit 200 has an opening 204. The acoustic sensor 206 is arranged outside the conduit 200. The membrane 208 is arranged to cover the opening 204 to separate the acoustic sensor 206 and the airflow 202 from each other. The membrane 208 comprises an electro-active polymer 1000. The membrane 208 is adapted to deform in response to sound 212 propagating along the airflow 202. The electro-active polymer 1000 is adapted to generate an electric signal 1002 in response to a deformation of the membrane 208.

For example, the electric signal 1002 is transmitted to the processing system 318. The processing system 318 is configured to process the signal 1002. For example, the processing system 318 is configured to determine a frequency or an intensity of the sound 212 based on the electric signal 1002. For example, the processing system 318 is configured to implement an algorithm to classify the sound 212. For example, the processing system 318 is configured to implement an algorithm to derive information of the subject 104 from the sound 212. For example, the processing system 318 is configured to implement an algorithm to derive information of a component of the respiratory support device 100 from the sound 212. Optionally, the processing system 318 is arranged on a base plate 316, such as a printed circuit board (PCB).

Optionally, the membrane 208 comprises a base layer 804 and a protective layer 806, similar to the embodiment depicted in FIG. 8. The protective layer 806 is chemically bonded to the base layer 804. The protective layer 806 protects the base layer 804 from humidity in the airflow 202.

Optionally, the respiratory support device 100 comprises an air delivery tube 108 and a water reservoir 900, similar to the embodiment depicted in FIG. 9. The air delivery tube 108 is adapted to deliver the pressurized air 102 to a patient interface 106. The conduit 200 is pneumatically connected to the air delivery tube 108. The conduit 200 couples the water reservoir 900 and the air delivery tube 108 to each other.

Optionally, the respiratory support device 100 comprises an accelerometer 800 attached to the membrane 208, similar to the embodiment depicted in FIG. 8. The accelerometer 800 is adapted to vibrate in response to the sound 212 propagating along the airflow 202. The accelerometer 800 is adapted to generate a signal representative of the sound 212 based on a vibration of the accelerometer 800. In this optional embodiment, both the electric signal 1002 of the electro-active polymer 1000 and the signal 802 from the accelerometer 800 are transmitted to the processing system 318. The processing system 318 is configured to process both the electric signal 1002 from the electro-active polymer 1000 and the signal 802 from the accelerometer 800. For example, the processing system 318 is configured to determine a frequency or an intensity of the sound 212. For example, the processing system 318 is configured to implement an algorithm to classify the sound 212. For example, the processing system 318 is configured to implement an algorithm to derive information of the subject 104 from the sound 212. For example, the processing system 318 is configured to implement an algorithm to derive information of a component of the respiratory support device 100 from the sound 212.

Optionally, the membrane 208 comprises a silicone material.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to an advantage. Any reference signs in the claims should not be construed as limiting the scope.

## Claims

1. A respiratory support device (100) for providing pressurized air (102) to a subject (104), the respiratory support device (100) comprising:
a conduit (200) for conveying an airflow (202),
wherein the conduit (200) has an opening (204);
an acoustic sensor (206) arranged outside the conduit (200);
a membrane (208) arranged to cover the opening (204) to separate the acoustic sensor (206) and the airflow (202) from each other;
a body (210) attached to the membrane (208),
wherein the body (210) is adapted to vibrate in response to sound (212) propagating along the airflow (202),
wherein the acoustic sensor (206) is adapted to generate a signal (314) representative of the sound (212) based on a vibration of the body (210).

2. The respiratory support device (100) according to claim 1, wherein the acoustic sensor (206) is a microphone having a first part (311) and a second part (312),
wherein the microphone is adapted to generate the signal (314) based on a movement of the first part (311) and the second part (312) relative to each other,
wherein the body (210) comprises the first part (311).

3. The respiratory support device (100) according to claim 2, wherein the membrane (208) comprises a protrusion (500, 600) adapted to attach the body (210) to,
wherein the body (210) is attached to the protrusion (500, 600).

4. The respiratory support device (100) according to claim 3, wherein the membrane (208) comprises a silicone material,
wherein the protrusion (500, 600) comprises the silicone material.

5. The respiratory support device (100) according to any one of claims 3-4, wherein the protrusion (600) forms a receptacle (602) for receiving at least part of the first part (311).

6. The respiratory support device (100) according to any one of claims 3-4, wherein the protrusion (500) has a proximal part (502) and a distal part (504),
wherein the proximal part (502) has a different cross-section than the distal part (504),
wherein the first part (311) is arranged around the proximal part (502).

7. The respiratory support device (100) according to any one of claims 2-6, wherein the microphone is a dynamic microphone,
wherein the first part (311) comprises one of a magnet and a coil,
wherein the second part (312) comprises the other of a magnet and a coil,
wherein the magnet and the coil are arranged to cooperate electromagnetically with each other to generate an electrical signal (314) in response to the movement of the first part (311) and the second part (312) relative to each other.

8. The respiratory support device (100) according to claim 2-6, wherein the microphone is a condenser microphone,
wherein the first part (311) comprises a first metal body (411),
wherein the second part (312) comprises a second metal body (412),
wherein the first metal body (411) and the second metal body (412) form a capacitor having a capacitance,
wherein the condenser microphone is adapted to generate a change in capacitance in response to the movement of the first part (311) and the second part (312) relative to each other.

9. The respiratory support device (100) according to claim 1,
wherein the acoustic sensor (206) comprises a sensor membrane (700),
wherein the body (210) extends from the membrane (208) to the sensor membrane (700) (208),
wherein the acoustic sensor (206) is adapted to generate the signal representative of the sound (212) based on a vibration of the sensor membrane (700).

10. The respiratory support device (100) according to claim 1,
wherein the body (210) and the acoustic sensor (206) are integrated,
wherein the acoustic sensor (206) comprises an accelerometer (800).

11. The respiratory support device (100) according to any one of the preceding claims, wherein the membrane (208) comprises a base layer (804) and a protective layer (806),
wherein the body (210) is chemically bonded to the base layer (804),
wherein the protective layer (806) protects the base layer (804) from humidity in the airflow (202).

12. The respiratory support device (100) according to any one of the preceding claims, comprising a seal (216) and a casing (214),
wherein the acoustic sensor (206) is arranged inside the casing (214),
wherein the casing (214) has a casing opening (222) arranged to face the opening (204) in the conduit (200),
wherein the membrane (208) is attached to the casing (214),
wherein the seal (216) is adapted to provide a sealing engagement between the conduit (200) and the casing (214) by abutting a surface of the conduit (200) enclosing the opening (204) and by abutting a surface of the casing (214) enclosing the casing opening (222).

13. The respiratory support device (100) according to any one of the preceding claims, comprising
an air delivery tube (108); and
a water reservoir (900),
wherein the air delivery tube (108) adapted to deliver the pressurized air (102) to a patient interface (106);
wherein the conduit (200) is pneumatically connected to the air delivery tube (108);
wherein the conduit (200) couples the water reservoir (900) and the air delivery tube (108) to each other.

14. An acoustic sensor system (230) for use in the respiratory support device (100) according to any one of the preceding claims, wherein the acoustic sensor system (230) comprises:
the acoustic sensor (206);
the membrane (208);
the body (210) attached to the membrane (208); and
a casing (214);
wherein the acoustic sensor (206) is arranged inside the casing (214),
wherein the casing (214) has a casing opening (222) arranged to face the opening (204) in the conduit (200),
wherein the membrane (208) is arranged to cover the casing opening (222).

15. A membrane (208) comprising a protrusion (500, 600) for use in the respiratory support system according to any one of claims 3-6.
